(19) Europäisches Patentamt
European Patent Office
Office européen des brevets

(11) Publication number: **0 117 689 A1**

(12) **EUROPEAN PATENT APPLICATION**

(21) Application number: 84301025.7

(22) Date of filing: 17.02.84

(51) Int. Cl.³: G 01 N 33/72, G 01 N 33/52, C 12 Q 1/28

(30) Priority: 01.03.83 US 471113

(43) Date of publication of application: 05.09.84 Bulletin 84/36

(84) Designated Contracting States: AT BE CH DE FR GB IT LI LU NL SE

(71) Applicant: SMITHKLINE DIAGNOSTICS, INC., 485 Potrero Avenue, Sunnyvale California 94086 (US)

(72) Inventor: Lawrence, Paul Joseph, 2082 Abbey Lane, Campbell California 95008 (US)
Inventor: Townsley, Charles William, 781 Foxridge Place, San Jose California 95133 (US)

(74) Representative: Johnston, Walter Paul et al, Patent Department SMITH KLINE & FRENCH LABORATORIES LTD Mundells, Welwyn Garden City Hertfordshire AL7 1EY (GB)

(54) **Improved specimen slide for occult blood testing.**

(57) A specimen test slide having a front panel and a rear panel. The front panel has a plurality of openings. A guaiac sheet containing haptoglobin on a portion thereof underlies these openings. A hinged cover overlies the openings. The rear panel has flap means opposite said openings which is pivotable to expose the underside of the sheet to permit application of a developing solution. The haptoglobin portion of the sheet upon contact with hemoglobin forms a complex which reacts with guaiac in the presence of an acid buffered developing solution to dye said portion blue.

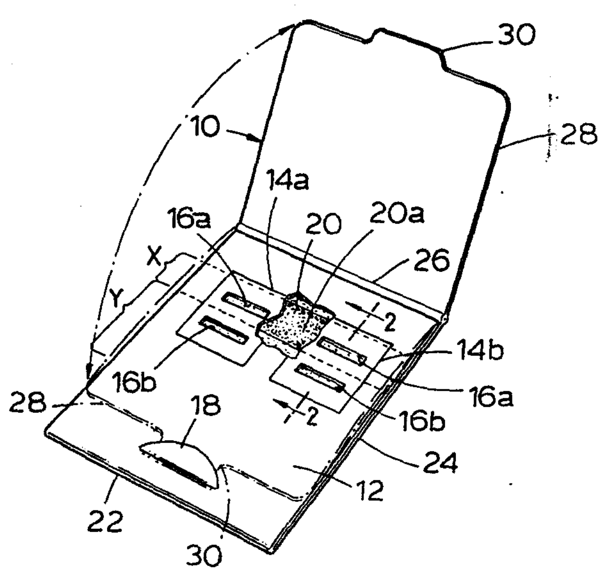


EP 0 117 689 A1

SMITHKLINE BECKMAN CASE 14143

# IMPROVED SPECIMEN SLIDE FOR OCCULT BLOOD TESTING

Specimen test slides and procedures for detecting occult blood in fecal matter are well known. For example, U. S. Patent No. 3,996,006 discloses slides having a substrate consisting of a specimen receiving sheet between a front panel and a rear panel with one or more openings in the front panel and an opening in the rear panel and pivotal covers to cover these openings. Typically, in the case of a test for occult blood in feces, the specimen receiving sheet is absorbent paper impregnated or printed with guaiac and a developing solution such as a peroxide solution is applied through the opening in the rear panel. One such test slide is sold under the trademark of "Hemoccult".

Briefly, the test procedure is as follows. A sample of fecal matter is smeared onto the guaiac paper through an opening of the front panel. The panel is then covered and the flap of the rear panel is opened. A developing solution such as hydrogen peroxide is applied to the guaiac paper via the corresponding opening in the rear panel. If blood is present in the fecal matter, the guaiac reaction will color the paper blue. The blue color is due to the hemoglobin catalyzed oxidation of the guaiac.

$$\underset{\text{Colorless}}{\text{Guaiac}} + H_2O_2 \xrightarrow{\text{Hemoglobin}} \underset{\text{Blue Color}}{\text{"Oxidized" Guaiac}}$$

Unfortunately, this test is not specific for blood because many other substances which may be present in fecal samples, for example, inorganic metals such as copper or iron, plant peroxidases and cytochromes, can catalyze the same reaction and produce a positive test result. It is very difficult to identify the actual catalyst present in a given fecal sample which produces a positive guaiac

test. Due to these factors, approximately four percent of the fecal blood tests result in what is known as false positive reactions. No physiological lesion can be found in these false positive tests and they add significantly to the cost of screening programs. The medical follow up tests are expensive and inconvenient. The sensitivity of guaiac based tests can be decreased to minimize the incidence of false positive test results but this increases the probability of failure to detect small quantities of blood in a given sample, i.e., false negative results. Alternatively, such sensitivity can readily be increased but the incidence of false positive tests would also increase. It is for these reasons that a guaiac based test specific for fecal hemoglobin is badly needed.

Another limiting characteristic of the present guaiac test is that the hemoglobin catalyzed blue color formation is pH dependent. The developing solution does not operate reproducibly below a certain pH. The pH range for the successful performance of the above known system is from about 4.0 to 7.0 with the optimum pH being from 5.5 to 6.0. Thus, if hemoglobin is applied to the guaiac treated substrate in a medium that differs significantly from the optimum pH, little or possibly no color will be detected.

A still further disadvantage of the present commercially available specimen test slides is the poor readability. The blue color which develops during a positive reaction, particularly at low hemoglobin concentrations, disappears rapidly. In some instances the blue color is barely detectable.

It is therefore the object of this invention to improve the performance of specimen occult blood test slides such as "Hemoccult" in the critical areas of specificity, readability and sensitivity, while still maintaining the simple, rapid and inexpensive testing procedures and development.

Unexpectedly, it was discovered that when

hemoglobin was complexed with a protein such as, for example, haptoglobin on a specimen test slide containing a reagent-paper matrix, the pseudo-peroxidease activity was greatly enhanced under defined developmental conditions. This on-slide detection of hemoglobin resulted in greater specificity, readability, and sensitivity of the slide. It was further discovered that conditions such as pH, proper developing solutions and geometry of the openings are necessary for the proper performance of the test.

The "immunological-like" detection of hemoglobin is accomplished by binding hemoglobin with a protein such as, for example, haptoglobin or other hemoglobin specific binding proteins or immunoglobin. Haptoglobin, which is a serum $alpha_2$ glycoprotein, and binds hemoglobin in an almost irreversible manner with high specificity is advantageously employed. Haptoglobin, unlike hemoglobin, does not catalyze the oxidation of guaiac. Therefore, the addition of a developer to the hemoglobin/haptoglobin complex on a guaiac impregnated support produces a color which is dependent on hemoglobin.

In addition, haptoglobin has the overriding advantage in that it protects the hemoglobin catalytic activity under conditions where hemoglobin by itself does not show, or shows less guaiac activity, such as low pH ranges.

The pH is a very critical aspect of this invention and is one of the conditions necessary for the proper performance of the test. The catalytic activity of hemoglobin is affected at a low pH, around 4, and fails to produce a positive guaiac test result. However, when hemoglobin is complexed with haptoglobin, the catalytic activity is protected and it produces a positive test in the presence of guaiac and hydrogen peroxide. This test is therefore primarily specific for hemoglobin. If the pH is too low, the complex will be destroyed. If the pH is too high, a blue color, positive test, will result with hemoglobin and guaiac only. It is therefore important to

produce a pH environment wherein the catalytic activity o hemoglobin by itself will be inactivated and not result i a positive reaction but the catalytic activity of the hemoglobin-haptoglobin complex will not be destroyed and give a positive reaction. The pH range discovered for the successful performance of this test is from about 2 to 5. Preferably the pH range is from 2.5 to 3.5 and most advantageously about 2.9.

In order to maintain the proper pH, the developing solution must be buffered.

Unlike the developing solutions employed for the commercial fecal occult blood testing slides which are alcoholic peroxide solutions, the developing solutions of this invention are aqueous peroxide solutions. It is wel known that hemoglobin is bound nonspecifically to filter paper impregnated with guaiac and that removal of hemoglobin from the paper is extremely difficult. However, the formation of the hemoglobin/haptoglobin complex together with the aqueous developer makes the hemoglobin much more soluble in the reagent paper matrix and therefore permits the hemoglobin to migrate or flow on the paper. This migration does not occur if the standard alcoholic-peroxide developing solutions are employed.

It has further been discovered that a deeper and more stable blue color is developed if the peroxide content is kept low in the aqueous solution. The commercial alcoholic-peroxide developing solutions contain about 5% peroxide. The solutions of this invention contain from about 0.01% to 1%, preferably about 0.04% peroxide.

The following acetate/citrate buffered developer solution is advantageously employed.

| Ingredients | | Range |
|---|---|---|
| Acetate Buffer | | 0.01 M to 0.50 M |
| Citrate Buffer | | 0.01M to 0.50M |
| Pluronic F 108 | | 0.05% to 2% (w/v) |
| Hydrogen peroxide | | 0.01% to 0.5% (w/w) |
| Water | q.s. | to 100 ml. |
| pH | | 2 to 5 |

Other universal buffered systems such as acetate alone, citrate alone, phthalate, citrate-phosphate, may be employed in the developer solution of this invention. Non-ionic detergents such as Pluronics, Triton, Brij 300/35 and Tweens may be added to the buffered developer in order to increase the sensitivity of the test. Most advantageously pluronic F 108, an ethylene oxide condensate is employed.

The geometry of the openings that receive the fecal samples also plays an important role in this invention. Preferably the openings should be narrow. These openings insure a maximum flow of developer through the specimen per amount of sample and therefore permit a more rapid diffusion or migration of hemoglobin. This results in a more intense blue color which is spread over a greater area making the results much easier to read. The openings also control the size of the specimen area and the amount of specimen to be applied in each opening, i.e., approximately the same amount is tested in each opening. This therefore allows for a more controlled test.

Preferably the width of the openings will be from about 1/16 to about 1/8 of an inch, advantageously from about 1/16 to about 3/32 of an inch. There are no distinct technical advantages for a maximum or minimum length. As the length is increased the openings become too large for the slide or amount of stool sampled. Optimally, the length is between 1/2 and 3/4 of an inch.

A detailed description and better understanding of this invention can be had by referring to the accompanying drawings which show a preferred embodiment of the present invention.

Figure 1 is a perspective view of the slide in an open position.

Figure 2 is an enlarged fragmentary sectional view taken on line 2,2 of Figure 1.

Figure 3 is an enlarged fragmentary sectional view of area enclosed by dot-dash circle of Figure 2.

Figure 4 is a perspective view of the reverse side of the slide shown in Figure 1 exposing the testing surface including the monitors.

Figure 5 is an enlarged fragmentary sectional view taken on line 5,5 of Figure 4.

Figure 6 is an enlarged plan rear view of the slide showing developing solution being applied to the testing surface.

Figure 7 is an enlarged plan rear view of the slide showing developing solution being applied to the monitor area.

Figure 8 is an enlarged plan rear view of the testing surface showing a positive reaction.

Figure 9 is an enlarged plan rear view of the testing surface showing results of a slide with hemoglobin and impurities.

Figure 10 is an enlarged plan rear view of the testing surface showing a suspect test which should be repeated.

Referring to Figure 1, test slide 10 has a front panel 12 that includes two separate fecal sample receiving Zones, X and Y, with each Zone having a pair of adjacent upper and lower openings 16 a and 16 b. The front panel 12 overlies an intermediate absorbent sheet 20 having a portion thereof treated with haptoglobin 20 a. The front panel 12 makes a first fold along its lower terminal edge 22 forming a rear panel 24. The absorbent sheet 20 is retained between front panel 12 and rear panel 24. The rear panel terminates in a hinge portion 26 joining front cover panel 28 having a locking tab 30 which engages slot 18. As illustrated in Figures 2 and 3 the portion of the absorbent sheet 20 underlying Zone X and the upper openings 16 a is layered with haptoglobin 20 a and guaiac 20 b. The portion of the absorbent sheet underlying Zone Y and the lower openings 16 b is not treated with haptoglobin and contains guaiac only.

As shown in Figure 4, the testing surface 32 has

a control area 36 containing monitors 36 a and 36 b and barrier strips 38.

Figure 5 demonstrates that the absorbent paper 20 underlying the positive monitor 36 a is treated with a layer of hemoglobin 20 c and haptoglobin 20 a. Negative monitor 36 b also has hemoglobin present but is free of haptoglobin. Neither monitor has guaiac present. The absence of guaiac in the control area insures better stability for the hemoglobin which is applied to the area.

To use the slide, the patient applies, with an applicator, a thin smear of specimen from separate portions of stool to absorbent sheet 20 through openings 16 a and 16 b. The front cover 28 is then closed by locking tab 30 in slit 18. The patient returns the slide either to his physician or a laboratory. The physician or technician pulls flap 34 free of rear panel 24 and opens it outwardly. Through the opening thus made, the developing solution 44 is applied to testing surface 32 between openings 16 a and 16 b. As viewed in Figures 6 and 7, the solution spreads outwardly 46 over the fecal stains 42. The solution is prevented from contaminating the control area by barrier strips 38.

Due to the increased solubility of the hemoglobin-haptoglobin complex, the complex also spreads to the top of the absorbent paper strip. The developing solution is then added to the control area as noted in Figure 7.

The test results are then observed. A blue color denotes a positive reaction. If the blue color in the area behind Zone X and openings 16 a is greater than the color in Zone Y, this denotes a positive test. If there is no blue color in either area behind Zone X or Y, the test is negative. If a blue color of equal intensity develops behind Zones X and Y and the openings of 16 a and 16 b, this indicates contamination with interfering substances such as, for example, plant peroxidases, hemin, hematin, iron and copper salts. In this case the test

should be repeated.

A positive result is indicated in Figure 8. A blue color 48 is noted behind Zone X and openings 16 a of the slide. This area which has haptoglobin present forms the hemoglobin-haptoglobin complex. The catalytic activity of the hemoglobin is thus protected from the pH of the developer and reacts with the guaiac to form the blue color. The bottom portion of the testing area which contains no haptoglobin is colorless because the catalytic activity of hemoglobin was inactivated by the pH environment and therefore could not react with the guaiac.

In regard to the control area 36, the solution migrates the hemoglobin present in monitor 36 a above and outside the control area where guaiac is present to react with the complex and form a blue color. Since the portion outside and below the monitor 36 b is not treated with haptoglobin, the hemoglobin is once again degraded and cannot react with the guaiac to give a blue color.

Figure 9 shows a positive test with perhaps some impurities present. The blue color behind Zone A and openings 16 a is deeper than the color around Zone B which may have a small degree of contamination or an excess of hemoglobin may have migrated to the zone.

Figure 10 represents a test that should be repeated because of contaminants. All areas are the same shade of blue.

The ability to be specific and to reject those tests where excessive contaminants do not allow a clear decision is the main advantage of the present slide and test.

When a guaiac support such as absorbent paper containing hemoglobin is treated with haptoglobin, the catalytic activity of the resulting hemoglobin/haptoglobin complex is from about five to fifty times greater than hemoglobin alone under the specified conditions. Further, this increase in activity is accomplished with a significant decrease of false positives.

Readability of the slide is also improved. The blue color which denotes a positive test remains for about five minutes in contrast to the commercial slides in which the color disappears rapidly.

The above embodiments are illustrative and are not intended to be limiting. For example, the control area 36 can be positioned on other isolated areas of the absorbent paper at some distance from the portions of the paper underlying openings 6 a and 6 b. The control area may also be positioned on a portion of the sheet facing the front panel, i.e., below fecal sample receiving zone.

A further embodiment could include a test sheet impregnated with guaiac having haptoglobin printed thereon in place of the layered sheet as shown in Figure 3.

Claims :-

1. An occult blood specimen test slide having a front panel, a rear panel, said front panel having a plurality of openings, a guaiac treated sheet means between the front and rear panels underlying each of said openings and having a portion of said sheet containing haptoglobin, a hinged cover adapted to overlie a portion of the front panel and said openings, and flap means in the rear panel opposite said openings and pivotable to expose the underside of the sheet; said haptoglobin having the ability to complex hemoglobin and said complex having the ability to react with guaiac in the presence of an aqueous buffered developing solution to dye the haptoglobin portion of the slide blue.

2. The slide of Claim 1 in which the developing solution is buffered at a pH of from about 2 to 5.

3. An occult blood specimen test slide having a front panel, a rear panel, said front panel having a plurality of openings, a guaiac treated sheet means between the front and rear panels underlying each of said openings and having a portion of said sheet containing haptoglobin, a hinged cover adapted to overlie a portion of the front panel and said openings, a control area positioned on a portion of the sheet means isolated from the openings in the front panel, said area including a positive and negative monitor, and flap means in the rear panel opposite said openings and pivotable to expose the underside of the sheet; said haptoglobin having the ability to complex hemoglobin and said complex having the ability to react with guaiac in the presence of an aqueous buffered solution to dye the haptoglobin portion of the slide blue.

4. The slide of Claim 3 in which the positive and negative monitors include hemoglobin and said positive monitors additionally include haptoglobin.

5. The slide of Claim 3 in which the control area is positioned on a portion of the sheet facing the

rear panel.

6. A guaiac absorbent substrate for use in determining the presence of occult blood, said substrate having a portion thereof containing haptoglobin with the ability to complex hemoglobin and said complex having the ability to react with guaiac in the presence of an aqueous buffered developing solution to dye said portion blue.

7. The substrate of Claim 6 in which the developing solution is buffered at a pH of from about 2 to 5.

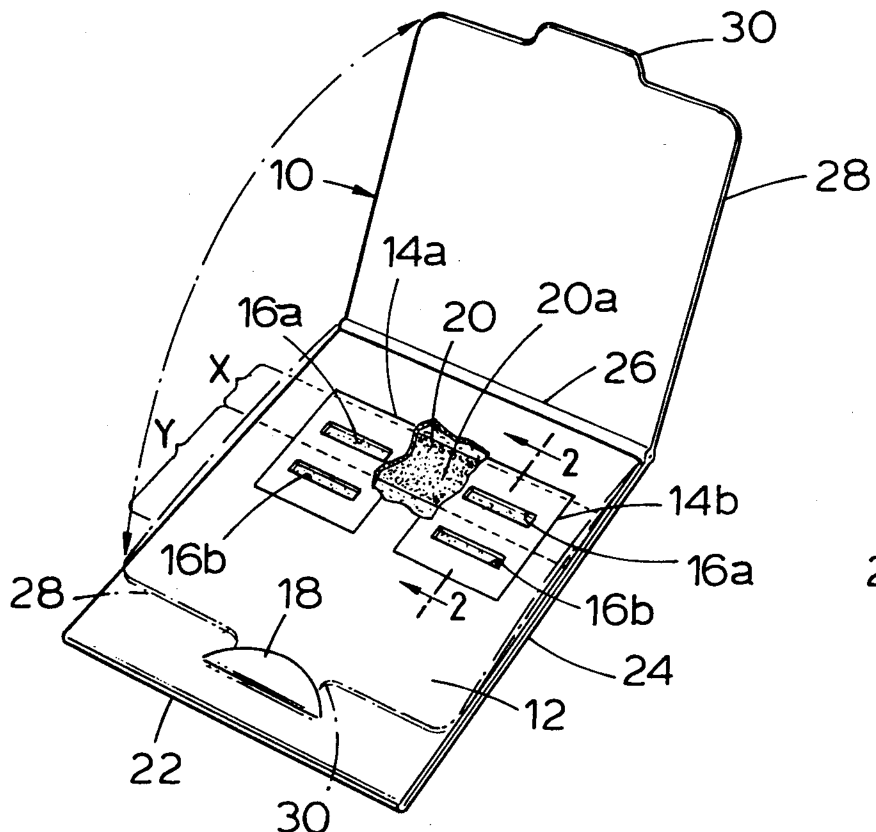

Fig.1

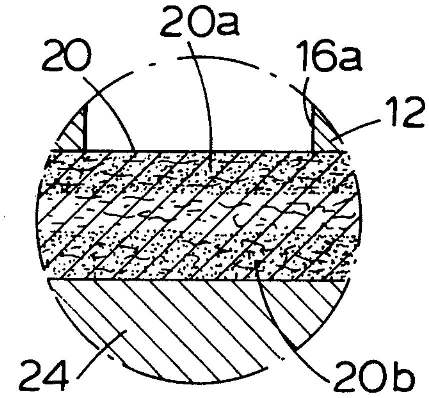

Fig.3

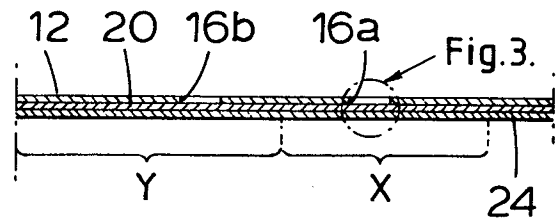

Fig.2

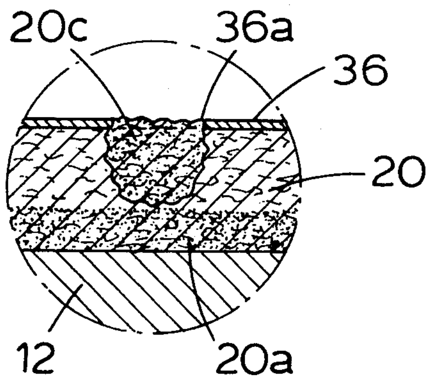

Fig.5

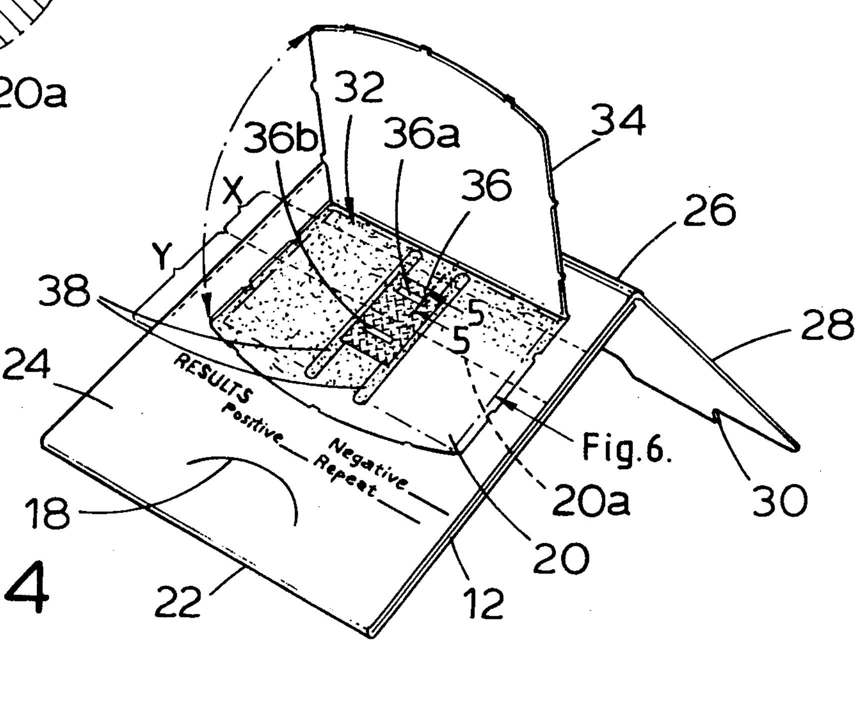

Fig.4

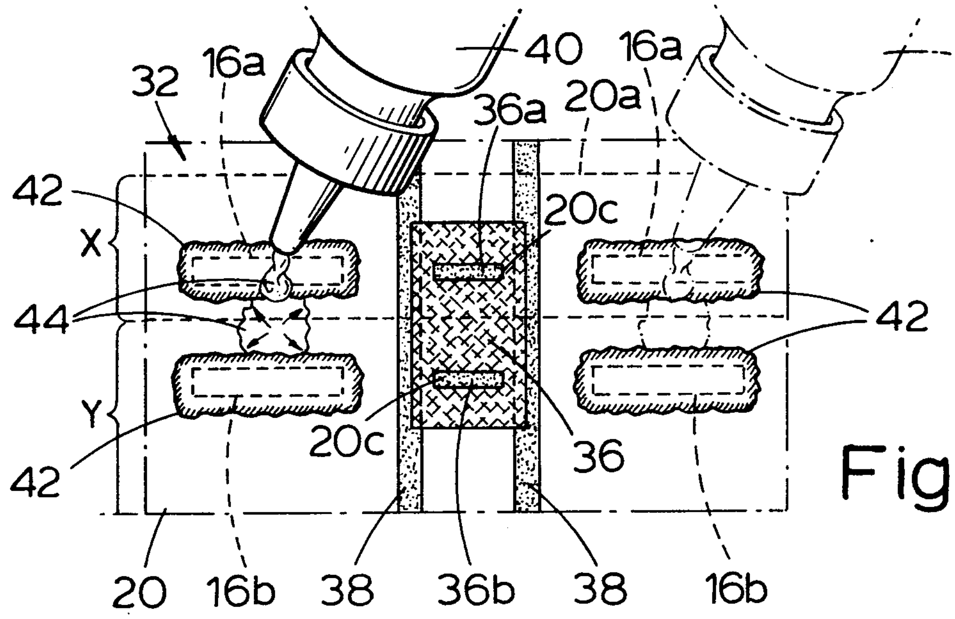


Fig. 6

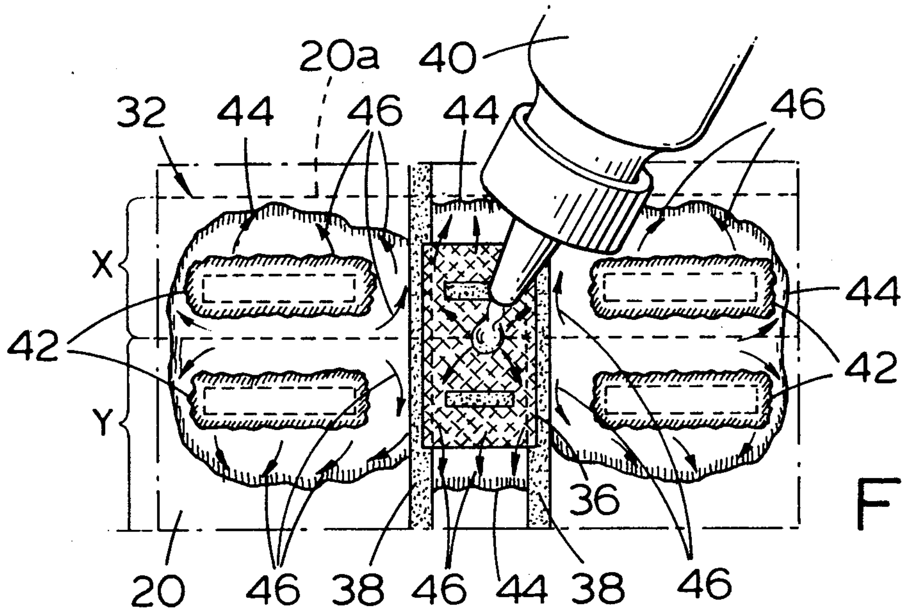


Fig. 7

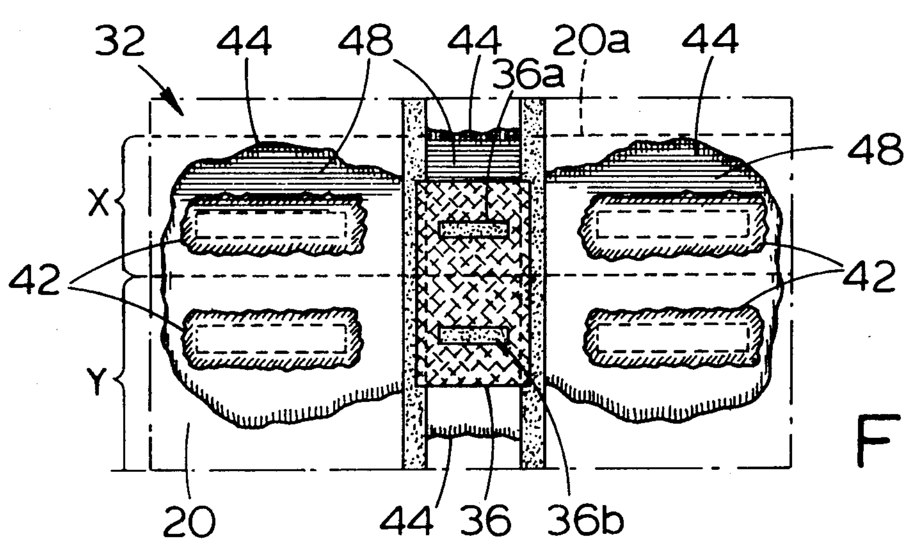


Fig. 8

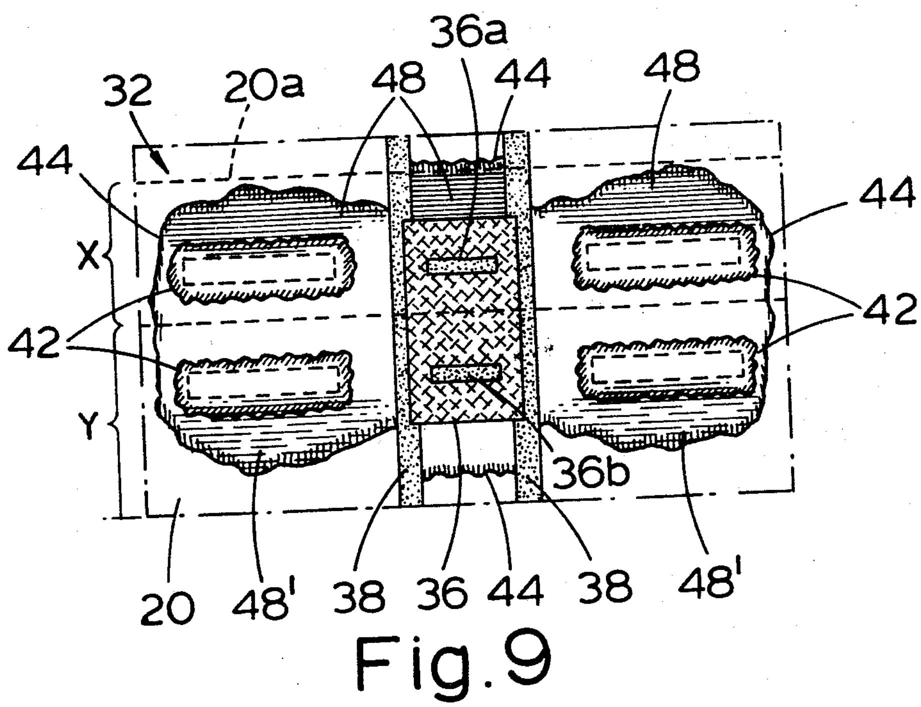
36a
48
44
48
32
20a
44
44
X
42
42
Y
36b
20
48'
38
36
44
38
48'

Fig. 9

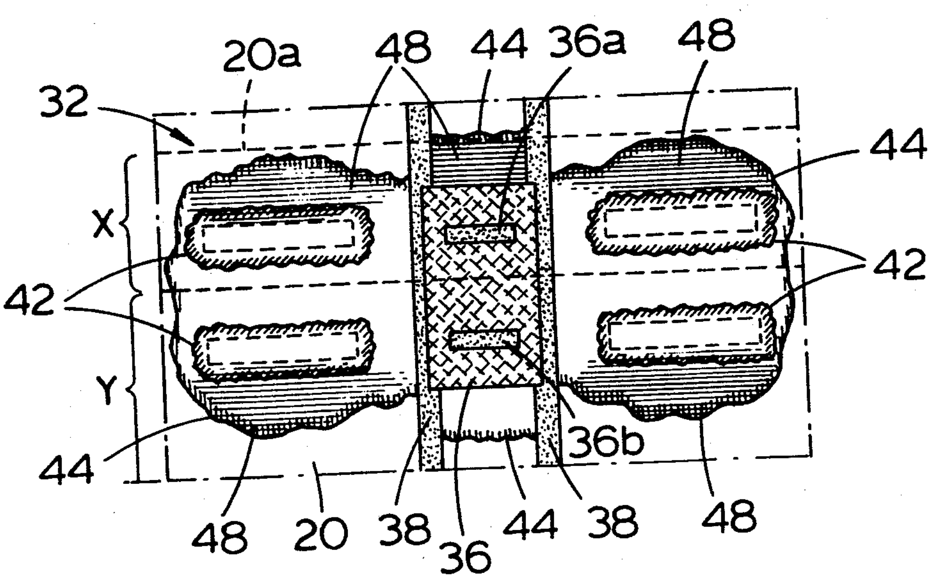
20a
48
44
36a
48
32
44
X
42
42
Y
36b
44
48
20
38
36
44
38
48

Fig. 10

0117689

European Patent Office

# EUROPEAN SEARCH REPORT

Application number
EP 84301025.7

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP - A1 - 0 064 392 (SMITHKLINE INSTRUMENTS INCORPORATED)<br>* Abstract * | 1,3 | G 01 N 33/72<br>G 01 N 33/52<br>C 12 Q 1/28 |
| A | US - A - 4 225 557 (HARTL et al.)<br>* Claims * | 1,3 | |
| D,A | US - A - 3 996 006 (PAGANO)<br>* Abstract * | 1,3 | |
| A | US - A - 4 175 923 (FRIEND)<br>* Abstract * | 1,3 | TECHNICAL FIELDS SEARCHED (Int. Cl. 3) |
| A | US - A - 4 092 120 (SUOVANIEMI et al.)<br>* Abstract * | 1,3 | G 01 N 33/00<br>C 12 Q |

The present search report has been drawn up for all claims

| Place of search | Date of completion of the search | Examiner |
|---|---|---|
| VIENNA | 15-05-1984 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82